# EUROPÄISCHE PATENTSCHRIFT

(11) **EP 0 742 190 B1**
(45) Veröffentlichungstag und Bekanntmachung des Hinweises auf die Patenterteilung: **23.12.1998**
(21) Anmeldenummer: 96106609.9
(22) Anmeldetag: 26.04.1996
(51) Int. Cl.: C07C 2/66, C07C 15/16

(54) **Verfahren zur Herstellung von Diarylethanen**
Process for the preparation of diarylethanes
Procédé de préparation de diaryléthanes

(30) Priorität: 06.05.1995 DE 19516717
(43) Veröffentlichungstag der Anmeldung: 13.11.1996
(73) Patentinhaber: BASF AKTIENGESELLSCHAFT, 67056 Ludwigshafen (DE)
(72) Erfinder: Gehrer, Eugen, Dr., 67069 Ludwigshafen (DE); Massonne, Klemens, Dr., 67368 Westheim (DE); Gausepohl, Hermann, Dr., 67112 Mutterstadt (DE); Fischer, Martin, Dr., 67071 Ludwigshafen (DE); Rieker, Christopher William, Dr., 68163 Mannheim (DE); Siggel, Lorenz, Dr., 69121 Heidelberg (DE)

(56) Entgegenhaltungen:
- EP-A- 0 015 758
- FR-A- 1 281 757
- PATENT ABSTRACTS OF JAPAN vol. 015, no. 313 (C-0857), 9.August 1991 & JP-A-03 118336 (KUREHA CHEM IND CO LTD), 20.Mai 1991,

## Beschreibung

Die vorliegende Erfindung betrifft eine Verfahren zur Herstellung von Diarylethanen durch Umsetzung von Aromaten mit Styrolen in Gegenwart eines Beta-Zeolithen oder eines Mordeniten als Heterogenkatalysator.

Die Herstellung von Diphenylethan durch Umsetzung von Aromaten mit Styrol(-derivaten) ist in JP63-238028 (1988) unter Verwendung von Y-Zeolithen, in Polymer Journal, Vol. 12, No. 6, Seite 407 (1980) unter Verwendung von Nafion, Amberlyst 15 oder CF₃SO₃H in JP-A-3 118 336 unter Verwendung von bestimmten, Schwermetall-ausgetauschten HY-Zeolithen, in FR-A-1 281 757 unter Verwendung von Säure-behandelten Mortmorilloniten sowie in EP-A-421 340 unter Verwendung von L-Zeolithen beschrieben. Die beschriebenen Synthese liefern nur beim Einsatz von alkylierten Aromaten brauchbare Ausbeuten, die jedoch zu wünschen übrig lassen. Bei der Verwendung von Benzol als Aromat entstanden überwiegend Oligomere des Styrols.

Der vorliegenden Erfindung lag daher die Aufgabe zugrunde, den zuvor genannten Nachteilen abzuhelfen.

Demgemäß wurde ein neues und verbessertes Verfahren zur Herstellung von Diarylethanen der allgemeinen Formel I 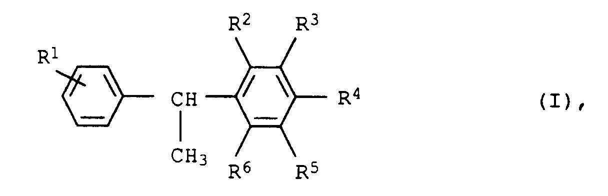 in der R¹, R², R³, R⁴, R⁵ und R⁶ Wasserstoff oder C₁- bis C₈-Alkyl bedeuten, durch Umsetzung von Aromaten der allgemeinen Formel II 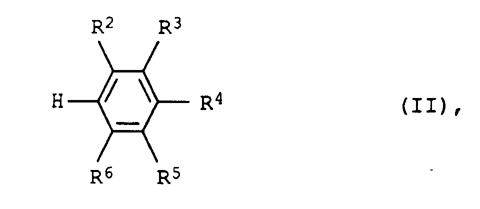 in der R², R³, R⁴, R⁵ und R⁶ die oben genannten Bedeutungen haben, mit Styrolen der allgemeinen Formel III 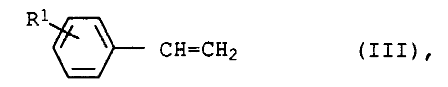 in der R¹ die oben genannten Bedeutungen hat, bei Temperaturen von (-20) bis 250°C und Drücken von 1 bis 100 bar in Gegenwart von Heterogenkatalysatoren gefunden, welches dadurch gekennzeichnet ist, daß man als Heterogenkatalysatoren Beta-Zeolithe oder Mordenite einsetzt.

Das erfindungsgemäße Verfahren läßt sich wie folgt durchführen:

Die Umsetzung der Aromaten II mit den Styrolen III kann diskontinuierlich oder kontinuierlich bei Temperaturen von (-20) bis 250°C, bevorzugt 80 bis 250°C, besonders bevorzugt 80 bis 200°C und Drücken von 1 bis 100 bar, bevorzugt 1 bis 30 bar, besonders bevorzugt bei Normaldruck (Atmosphärendruck) in Gegenwart eines Heterogenkatalysators, bevorzugt in flüssiger Phase durchgeführt werden.

Als Heterogenkatalysatoren eignen sich in der Regel stark saure, großporige Zeolithe wie Beta-Zeolithe oder Mordenite. Der Katalysator kann in der Regel entweder in Pulverform im Reaktionssystem suspendiert werden oder als Formkörper im einem Festbettreaktor eingesetzt werden.

Beta-Zeolithe sind beispielsweise aus der US-A-3 308 069 bekannt. Sie lassen sich mittels Tetraethylammoniumhydroxid bei Temperaturen im Bereich von 100 bis 150°C aus Gelen mit der Zusammensetzung TEA₂O:SiO₂:Al₂O₃:Na₂O:H₂O kristallisieren, wobei das SiO₂/Al₂O₃-Verhältnis im Bereich von 10:1 bis 200:1, das Verhältnis Na₂O/TEAOH von 0 bis 1:1, TEAOH/SiO₂ von 0,1:1 zu 1:1 und H₂O/TEAOH von 20:1 bis 75:1 liegen kann. Sie besitzen ein großporiges, dreidimensionales Porensystem mit 12-gliederigen Ringen mit Durchmessern von 6,5 x 5,6 und 7,5 x 5,7 Å.

Die Reaktion wird in der Regel bevorzugt unter Bedingungen durchgeführt, die nur eine geringe Konzentration von Styrol zulassen. Dies kann durch den Einsatz von verdünnten Styrol-Lösungen oder durch das langsame Zudosierung des Styrols zum Reaktionssystem erfolgen. Eine besonders bevorzugte Methode zur diskontinuierlichen Herstellung besteht darin, daß man den Aromaten zusammen mit dem darin suspendierten Katalysator im einem Rührkessel vorlegt und das Styrol (oder eine Styrol-Lösung) während der Reaktion so langsam zutropft, daß sich ein Fließgleichgewicht zwischen dem zudosierten und dem abreagierenden Styrol auf einem niedrigen Konzentrationsniveau einstellt. Die stationäre Konzentration an Styrol sollte 3%, bevorzugt 0,1%, besonders bevorzugt 0,05% nicht überschreiten.

Eine weitere besonders bevorzugte Methode zur kontinuierlichen Herstellung besteht darin, daß man einen Rührkessel mit Überlauf verwendet, den Katalysator als Suspension im jeweiligen Aromaten im Rührkessel vorlegt und anschließend kontinuierlich ein Styrol/ Aromat-Gemisch so zudosiert, daß sich eine niedrige stationäre Styrolkonzentration einstellt. Die Konzentration an Styrol sollte 3%, bevorzugt 0,1%, besonders bevorzugt 0,05% nicht überschreiten. Das am Überlauf austretende Reaktionsgemisch kann mit einem Settler oder einem Filter vom Katalysator abgetrennt werden. Der Katalysator kann, gegebenenfalls nach einer Regenerierung, in die Reaktion zurückgeführt werden.
Die Diphenylethane I können über den Sumpf einer Destillationskolonne erhalten werden.

Die Substituenten R¹, R², R³, R⁴, R⁵ und R⁶ in den Verbindungen I, II und III haben folgende Bedeutungen und R¹ in ortho-, meta- oder para-Stellung stehen kann:
- Wasserstoff,
- C₁- bis C₈-Alkyl, bevorzugt C₁- bis C₄-Alkyl wie Methyl, Ethyl, n-Propyl, iso-Propyl, n-Butyl, iso-Butyl, sec.-Butyl, tert.- Butyl, besonders bevorzugt Methyl und Ethyl.

Die Verbindungen I, II und III, in denen die Substituenten R¹, R², R³, R⁴, R⁵ und R⁶ gleichzeitig Wasserstoff bedeuten, 1,1-Diphenylethan, Benzol und Styrol, sind besonders bevorzugt.

Diphenylethan ist ein wichtiges Zwischenprodukt für die Herstellung von Diphenylethen, welches als Comonomeres für die Herstellung von Kunststoffen verwendet wird. Diphenylethen kann durch katalytische Dehydrierung von Diphenylethan in guten Ausbeuten gewonnen werden.

### Beispiele

Zur Analyse der nachfolgenden Experimente wurde ein Gaschromatograph mit einer 30 m langen Kapillarsäule ( DB5, 0.1 um) verwendet. (Temperaturprogramm: 5 Minuten bei 60°C, 10°C/Minute bis 300°C, 15 Minuten Endtemperatur). Die Quantifizierung erfolgte mit Hilfe Toluol als innerem Standard.

Die drei entstandenen Dimere (Identifizierung mittels GC/MS) wurden in der folgenden Zusammenstellung zusammengezählt. Ihr Eichfaktor wurde mit dem für Diphenylethan gleichgesetzt. Die Eichfaktoren für Benzol (nicht aufgelistet), Styrol und Diphenylethan wurden über eine Konzentrationsreihe mit Toluol als mit internem Standard bestimmt.

### Beispiel 1

In einem 250 ml Dreihalskolben mit Rückflußkühler, Thermometer, Tropftrichter wurden 5 g β-Zeolith und 80 g Benzol vorgelegt und Rückfluß gekocht. Anschließend tropfte man langsam, im Verlauf von 2 Stunden eine Mischung von 10 g Styrol und 10 g Benzol zu.

Die Reaktionsmischung wurde danach mittels GC analysiert.

Das Produkt enthielt 15.6 % Diphenylethan, was einer Ausbeute von 89.3 % entspricht. (2.06 % Dimere, 0.02 % Trimer).

### Beispiele 2 bis 6 (Konzentrationsabhängigkeit)

In einem 50 ml Druckglasgefäß wurden 1 g β-Zeolith und 10 ml Styrol/Benzol-Lösung 5h lang bei 30°C gerührt. Das Reaktionsprodukt wurde anschließend mittes GC analysiert.

| Produktzusammensetzung | | | | | | |
|---|---|---|---|---|---|---|
| Beispiel Nr. | Styrolkonz. in Benzol [%] | Styrol [%] | Diphenylethan [%] | Dimere [%] | Trimere [%] | Ausbeute [%] |
| 2 | 1 | 0 | 1.37 | 0.11 | 0 | 78.3 |
| 3 | 2.5 | 0 | 1.78 | 0.84 | 0 | 40.7 |
| 4 | 5 | 0 | 1.96 | 2.27 | 0.1 | 22.4 |
| 5 | 10 | 0 | 1.77 | 4.85 | 0.2 | 10.4 |
| 6 | 20 | 0 | 1.56 | 9.03 | 0.3 | 4.5 |

### Beispiele 7 bis 10 (Temperaturabhängigkeit)

In einem 50 ml Druckglasgefäß mit Manometer, Rührfisch und Entspannungsventil wurden 1 g β-Zeolith und 10 ml einer Lösung von 10 % Styrol in 90 % Benzol 5 h lang bei der Temperatur T (siehe Tabelle) gerührt. Das Reaktionsprodukt wurde anschließend mittes GC analysiert.

| Produktzusammensetzung | | | | | | |
|---|---|---|---|---|---|---|
| Beispiel Nr. | Temperatur [°C] | Styrol [%] | Diphenylethan [%] | Dimere [%] | Trimere [%] | Ausbeute [%] |
| 7 | 0 | 0 | 1.7 | 4.81 | 0.3 | 9.7 |
| 8 | 30 | 0 | 1.77 | 4.85 | 0.2 | 10.1 |
| 9 | 50 | 0 | 1.86 | 4.63 | 0.3 | 10.6 |
| 10 | 100 | 0 | 1.59 | 5.37 | 0.1 | 9.1 |

### Beispiele 11 bis 14 (Zeolithkatalysatoren)

In einem 50 ml Glaskolben wurden 1 g Zeolith und 10 ml einer Lösung von 10 % Styrol in 90 % Benzol 5 h bei 30°C gerührt. Das Reaktionsprodukt wurde anschließend mittels GC analysiert.

| Produktzusammensetzung | | | | | | |
|---|---|---|---|---|---|---|
| Beispiel Nr. | Katalysator | Styrol [%] | Diphenylethan [%] | Dimere [%] | Trimere [%] | Ausbeute [%] |
| 11 | ZSM-5 | 2.26 | 0 | 2.42 | 0.05 | 0 |
| 12 | L-Zeolith | 9.13 | 0 | 0 | 0 | 0 |
| 13 | β-Zeolith | 0 | 1.77 | 4.85 | 0.2 | 10.1 |
| 14 | Mordenit | 0 | 1.59 | 3.27 | 0.1 | 6.1 |

### Beispiele 15 bis 17 (Temperaturabhängigkeit beim Semibatchverfahren)

In einem 250 ml Dreihalskolben mit Rückflußkühler, Thermometer, Tropftrichter wurden 5 g β-Zeolith und 80 g Benzol vorgelegt und bei der Temperatur T (siehe Tabelle) gerührt. Anschließend tropfte man langsam, im Verlauf von einer Stunde eine Mischung von 10 g Styrol und 10 g Benzol zu.

Die Reaktionsmischung wurde danach mittels GC analysiert.

| Produktzusammensetzung | | | | | | |
|---|---|---|---|---|---|---|
| Beispiel Nr. | Temperatur [°C] | Styrol [%] | Diphenylethan [%] | Dimere [%] | Trimere [%] | Ausbeute [%] |
| 15 | 30 | 1.03 | 2.4 | 5.07 | 0.05 | 13.7 |
| 16 | 50 | 2.37 | 1.92 | 4.48 | 0.02 | 11.0 |
| 17 | 80 | 0 | 13.81 | 3.61 | 0.26 | 78.9 |

### Beispiel 18 bis 20 (Katalysatormenge)

In einem 250 ml Dreihalskolben mit Rückflußkühler, Thermometer, Tropftrichter wurden x g β-Zeolith (siehe Tabelle) und 80 g Benzol vorgelegt und Rückfluß gekocht. Anschließend tropfte man langsam, im Verlauf von einer Stunde eine Mischung von 10 g Styrol und 10 g Benzol zu.

Die Reaktionsmischung wurde danach mittels GC analysiert.

| Produktzusammensetzung | | | | | | |
|---|---|---|---|---|---|---|
| Beispiel Nr. | Katalysatormenge [%] | Styrol [%] | Diphenylethan [%] | Dimere [%] | Trimere [%] | Ausbeute [%] |
| 18 | 0.1 | 9.33 | 0.07 | 1.18 | 0 | 1.3 |
| 19 | 1.0 | 0 | 6.7 | 7.4 | 0.14 | 38.3 |
| 20 | 5.0 | 0 | 13.81 | 3.61 | 0.26 | 78.9 |

### Beispiel 21 (Konzentrationsmaximum)

In einem 250 ml Dreihalskolben mit Rückflußkühler, Thermometer, Tropftrichter wurden 5 g β-Zeolith und 50 g Benzol vorgelegt und Rückfluß gekocht. Anschließend tropfte man langsam, im Verlauf von 3 Stunden 50 g Styrol zu.

Die Reaktionsmischung wurde alle 30 Minuten danach mittels GC analysiert.

| Produktzusammensetzung | | | | | |
|---|---|---|---|---|---|
| Reaktionszeit [min] | Styrol [%] | Diphenylethan [%] | Dimere [%] | Trimere [%] | Ausbeute [%] |
| 60 | 0 | 7.86 | 1.98 | 0 | 17.9 |
| 120 | 0 | 19.72 | 8.77 | 0.3 | 23.6 |
| 180 | 0.56 | 17.96 | 23.72 | 0.66 | 20.5 |

### Beispiel 22 bis 24 (Kontinuierliche Fahrweise)

10 g β-Zeolith (als 1 mm Stränge mit 10 % Pural) wurden in einen Rohrreaktor mit 8 mm Innendurchmesser und 1 m Länge gepackt. Der Rohrreaktor wurde auf die Temperatur T gebracht (40°C und 75°C in einem Heißluftofen, 8°C in einem gekühlten Ölbad) und kontinuierlich mit einer Lösung von 3 % Styrol in 97 % Benzol durchströmt. Die Produktausträge wurden in regelmäßigen Zeitabständen analysiert.

| Produktzusammensetzung | | | | | | | |
|---|---|---|---|---|---|---|---|
| Beispiel Nr. | Temperatur | Zeit [h] | Styrol [%] | Dipheny lethan [%] | Dimere [%] | Trimere [%] | Ausbeute [%] |
| 22 | 8 | 7 | 0 | 1.52 | 1.08 | 0.07 | 28.9 |
| | 8 | 54 | 0 | 0.72 | 1.98 | 0.06 | 13.7 |
| | 8 | 102 | 0 | 0.48 | 1.61 | 0 | 9.1 |
| 23 | 40 | 5 | 0 | 1.1 | 1.82 | 0 | 20.9 |
| | 40 | 42 | 0 | 0.6 | 1.28 | 0 | 11.4 |
| | 40 | 100 | 0 | 0.2 | 1.9 | 0 | 3.8 |
| 24 | 75 | 13 | 0 | 1.05 | 1.9 | 0 | 20 |
| | 75 | 42 | 0 | 0.61 | 1.34 | 0 | 11.6 |
| | 75 | 108 | 0 | 0.19 | 1.02 | 0 | 3.6 |

### Beispiel 25 (Reaktivdestillation)

Auf einen Dreihalskolben wird ein Fraktionieraufsatz und darüber ein Rückflußkühler gesetzt. Der Fraktionieraufsatz wird anstatt mit Füllkörpern mit 28.5 g (70 ml) β-Zeolithsträngen (1 mm) gefüllt. Im Dreihalskolben legt man eine Lösung von 20% Styrol in Benzol vor (20 g Styrol und 80 g Benzol) und kocht 3 h lang Rückfluß. Die Zusammensetzung des Sumpfes wurde alle 30 Minuten analysiert.

| Produktzusammensetzung | | | | |
|---|---|---|---|---|
| Reaktionszeit [Min] | Styrol [%] | Diphenylethan [%] | Dimere [%] | Trimere [%] |
| 30 | 28.1 | 1.35 | 0.84 | 0 |
| 60 | 11.8 | 15.0 | 8.0 | 0.2 |
| 90 | 8.9 | 17.58 | 10.7 | 0.3 |
| 120 | 6.9 | 20.42 | 13.4 | 0.34 |
| 150 | 4.5 | 25.26 | 17.1 | 0.45 |
| 180 | 2.84 | 27.99 | 19.57 | 0.54 |

## Patentansprüche

1. Verfahren zur Herstellung von Diarylethanen der allgemeinen Formel I 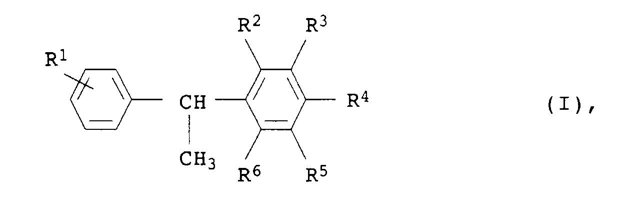 in der R¹, R², R³, R⁴, R⁵ und R⁶ Wasserstoff oder C₁- bis C₈-Alkyl bedeuten, durch Umsetzung von Aromaten der allgemeinen Formel II 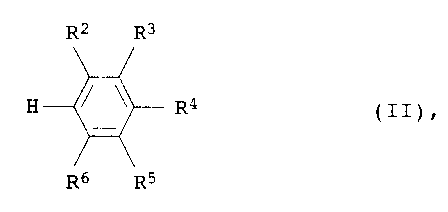 in der R², R³, R⁴, R⁵ und R⁶ die oben genannten Bedeutungen haben, mit Styrolen der allgemeinen Formel III 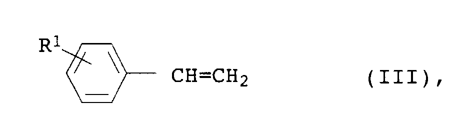 in der R¹ die oben genannten Bedeutungen hat, bei Temperaturen von (-20) bis 250°C und Drücken von 1 bis 100 bar in Gegenwart von Heterogenkatalysatoren, dadurch gekennzeichnet, daß man als Heterogenkatalysatoren Beta-Zeolithe oder Mordenite einsetzt.

2. Verfahren zur Herstellung von Diarylethanen I nach Anspruch 1, dadurch gekennzeichnet, daß R¹, R², R³, R⁴, R⁵ und R⁶ jeweils Wasserstoff bedeuten.

3. Verfahren zur Herstellung von Diarylethanen I nach den Ansprüchen 1 und 2, dadurch gekennzeichnet, daß man die Umsetzung bei Temperaturen von 80 bis 250°C durchführt.

4. Verfahren zur Herstellung von Diarylethanen I nach den Ansprüchen 1 und 2, dadurch gekennzeichnet, daß man die Umsetzung bei Temperaturen von 80 bis 200°C durchführt.

5. Verfahren zur Herstellung von Diarylethanen I nach den Ansprüchen 1 und 2, dadurch gekennzeichnet, daß man die Umsetzung bei Drücken 1 bis 30 bar durchführt.

6. Verfahren zur Herstellung von Diarylethanen I nach den Ansprüchen 1 und 2, dadurch gekennzeichnet, daß man die Umsetzung bei Normaldruck durchführt.

7. Verfahren zur Herstellung von Diarylethanen I nach den Ansprüchen 1 und 2, dadurch gekennzeichnet, daß man die Umsetzung mit einer stationären Styrol-Konzentration von nicht mehr als 3% durchführt.

8. Verfahren zur Herstellung von Diarylethanen I nach den Ansprüchen 1 und 2, dadurch gekennzeichnet, daß man die Umsetzung mit einer stationären Styrol-Konzentration von nicht mehr als 0,1% durchführt.

9. Verfahren zur Herstellung von Diarylethanen I nach den Ansprüchen 1 und 2, dadurch gekennzeichnet, daß man die Umsetzung mit einer stationären Styrol-Konzentration von nicht mehr als 0,05% durchführt.

## Claims

1. A process for preparing diarylethanes of the general formula I 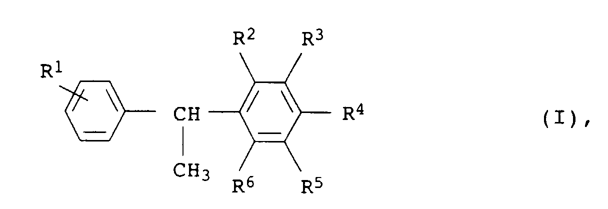 where R¹, R², R³, R⁴, R⁵ and R⁶ are each hydrogen or C₁-C₈-alkyl, by reacting aromatic compounds of the general formula II 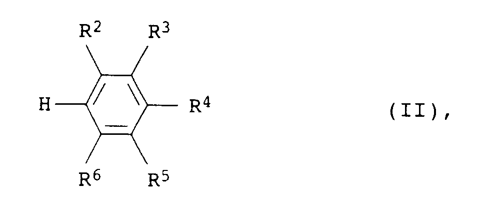 where R², R³, R⁴, R⁵ and R⁶ have the abovementioned meanings, with styrenes of the general formula III 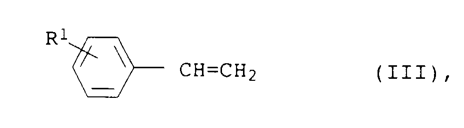 where R¹ has the abovementioned meanings, at from -20 to 250°C under from 1 to 100 bar in the presence of heterogeneous catalysts, wherein beta-zeolites or mordenites are used as heterogeneous catalysts.

2. A process for preparing diarylethanes I as claimed in claim 1, wherein R¹, R², R³, R⁴, R⁵ and R⁶ are each hydrogen.

3. A process for preparing diarylethanes I as claimed in claims 1 and 2, wherein the reaction is carried out at from 80 to 250°C.

4. A process for preparing diarylethanes I as claimed in claims 1 and 2, wherein the reaction is carried out at from 80 to 200°C.

5. A process for preparing diarylethanes I as claimed in claims 1 and 2, wherein the reaction is carried out under from 1 to 30 bar.

6. A process for preparing diarylethanes I as claimed in claims 1 and 2, wherein the reaction is carried out under atmospheric pressure.

7. A process for preparing diarylethanes I as claimed in claims 1 and 2, wherein the reaction is carried out with a stationary styrene concentration not exceeding 3%.

8. A process for preparing diarylethanes I as claimed in claims 1 and 2, wherein the reaction is carried out with a stationary styrene concentration not exceeding 0.1%.

9. A process for preparing diarylethanes I as claimed in claims 1 and 2, wherein the reaction is carried out with a stationary styrene concentration not exceeding 0.05%.

## Revendications

1. Procédé pour la préparation de diaryléthanes répondant à la formule générale I 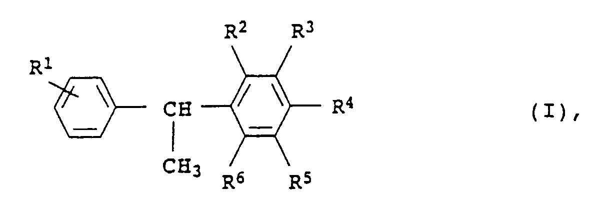 dans laquelle R¹, R², R³, R⁴, R⁵ et R⁶ représentent un atome d'hydrogène ou un groupe alkyle en C₁-C₈, par mise en réaction de composés aromatiques répondant à la formule générale II 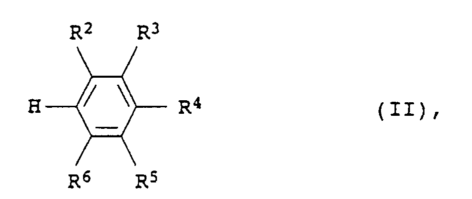 dans laquelle R², R³, R⁴, R⁵ et R⁶ ont les significations indiquées ci-dessus, avec des styrènes répondant à la formule générale III 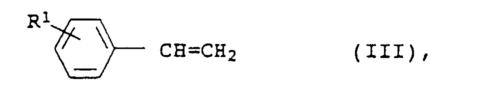 dans laquelle R¹ a les significations indiquées ci-dessus, à des températures de (-20) à 250°C et sous des pressions de 1 à 100 bar en présence de catalyseurs hétérogènes, caractérisé en ce qu'on met en oeuvre, à titre de catalyseurs hétérogènes, des zéolithes du type β ou des mordénites.

2. Procédé pour la préparation de diaryléthanes I selon la revendication 1, caractérisé en ce que R¹, R², R³, R⁴, R⁵ et R⁶ représentent respectivement un atome d'hydrogène.

3. Procédé pour la préparation de diaryléthanes I selon les revendications 1 et 2, caractérisé en ce qu'on effectue la mise en réaction à des températures de 80 à 250°C.

4. Procédé pour la préparation de diaryléthanes I selon les revendications 1 et 2, caractérisé en ce qu'on effectue la mise en réaction à des températures de 80 à 200°C.

5. Procédé pour la préparation de diaryléthanes I selon les revendications 1 et 2, caractérisé en ce qu'on effectue la mise en réaction sous des pressions de 1 à 30 bar.

6. Procédé pour la préparation de diaryléthanes I selon les revendications 1 et 2, caractérisé en ce qu'on effectue la mise en réaction sous pression normale.

7. Procédé pour la préparation de diaryléthanes I selon les revendications 1 et 2, caractérisé en ce qu'on effectue la mise en réaciion à une concentration stationnaire du styrène qui n'est pas supérieure à 3%.

8. Procédé pour la préparation de diaryléthanes I selon les revendications 1 et 2, caractérisé en ce qu'on effectue la mise en réaction à une concentration stationnaire du styrène qui n'est pas supérieure à 0,1%.

9. Procédé pour la préparation de diaryléthanes I selon les revendications 1 et 2, caractérisé en ce qu'on effectue la mise en réaction à une concentration stationnaire du styrène qui n'est pas supérieure à 0,05%.
